Europäisches Patentamt

European Patent Office

Office européen des brevets

(11) Publication number: **0 266 898 B1**

# EUROPEAN PATENT SPECIFICATION

(45) Date of publication of patent specification: **29.06.94**

(21) Application number: **87308741.5**

(22) Date of filing: **02.10.87**

(51) Int. Cl.[5]: **B01J 35/02**, B01J 23/46, B01J 23/74, B01J 23/89, B01J 23/76, B01J 23/56, C07C 1/04

(54) **Surface-supported particulate metal compound catalysts, their preparation and their use in hydrocarbon synthesis reactions.**

(30) Priority: **03.10.86 US 914781**
**07.05.87 US 46649**
**13.07.87 US 72517**

(43) Date of publication of application:
**11.05.88 Bulletin 88/19**

(45) Publication of the grant of the patent:
**29.06.94 Bulletin 94/26**

(84) Designated Contracting States:
**DE FR GB IT NL SE**

(56) References cited:
EP-A- 0 109 702     EP-A- 0 144 506
US-A- 2 606 159     US-A- 4 465 789
US-A- 4 542 122     US-A- 4 558 030
US-A- 4 568 663

(73) Proprietor: **EXXON RESEARCH AND ENGI-NEERING COMPANY**
**P.O.Box 390,**
**180 Park Avenue**
**Florham Park, New Jersey 07932-0390(US)**

(72) Inventor: **Behrmann, William Claus**
**546 Baird Drive**
**Baton Rouge Louisiana(US)**
Inventor: **Mauldin, Charles Harrison**
**11125 Suncrest Avenue**
**Baton Rouge Louisiana(US)**
Inventor: **Arcuri, Kym Brian**
**2028 Palmwood Drive**
**Baton Rouge Louisiana(US)**
Inventor: **Herskowitz, Mordechay**
**Hatzwi 69/19**
**Beer-Shava(IL)**

(74) Representative: **Somers, Harold Arnold et al**
**ESSO Engineering (Europe) Ltd.**
**Patents & Licences**
**Mailpoint 72**
**Esso House**
**Ermyn Way**
**Leatherhead, Surrey KT22 8XE (GB)**

**Description**

1. Field of the Invention

This invention relates to catalyst compositions, process wherein these compositions are used for the preparation of liquid hydrocarbons from synthesis gas, and process for the preparation of said catalyst. In particular, it relates to catalysts, and process wherein $C_{10+}$ distillate fuels, and other valuable products, are prepared by reaction of carbon monoxide and hydrogen over catalysts wherein a metal is dispersed as a thin film on the outside surface of a particulate carrier, or support, especially a titania carrier, or support.

2. Description of Information Disclosures

Particulate catalysts, as is well known, are normally formed by dispersing catalytically active metals, or the compounds thereof upon carriers, or supports. Generally, in making catalysts the objective is to disperse the catalytically active material as uniformly as possible throughout a particulate porous support, this providing a uniformity of catalytically active sites from the centre of a particle outwardly.

Catalysts have also been formed by dispersing catalytically active materials upon dense support particles; particles impervious to penetration by the catalytically active materials. Ceramic or metal cores have been selected to provide better heat transfer characteristics, albeit generally the impervious dense cores of the catalyst particles overconcentrates the catalytically active sites within a reduced reactor space and lessens the effectiveness of the catalyst. Sometimes, even in forming catalysts from porous support particles greater amounts of the catalytic materials are concentrated near the surface of the particles simply because of the inherent difficulty of obtaining more uniform dispersions of the catalytic materials throughout the porous support particles. For example, a catalytic component may have such strong affinity for the support surface that it tends to attach to the most immediately accessible surface and cannot be easily displaced and transported to a more central location within the particle. Catalyst dispersion aids, or agents are for this reason often used to overcome this effect and obtain better and more uniform dispersion of the catalytically active material throughout the catalyst particles.

Fischer-Tropsch synthesis for the production of hydrocarbons from carbon monoxide and hydrogen is now well known, and described in the technical and patent literature. The earlier Fischer-Tropsch catalysts were constituted for the most part of non-noble metals dispersed throughout a porous inorganic oxide support. The group VIII non-noble metals, iron, cobalt, and nickel have been widely used in Fischer-Tropsch reactions, and these metals have been promoted with various other metals, and supported in various ways on various substrates, principally alumina. Most commercial experience, however, has been based on cobalt and iron catalysts. The first commercial Fischer-Tropsch operation utilized a cobalt catalyst, though later more active iron catalysts were also commercialized. The cobalt and iron catalysts were formed by compositing the metal throughout an inorganic oxide support. The early cobalt catalysts, however, were of generally low activity necessitating a multiple staged process, as well as low synthesis gas throughout. The iron catalysts, on the other hand, are not really suitable for natural gas conversion due to the high degree of water gas shift activity possessed by iron catalysts.

U.S. 4,542,122 describes improved cobalt catalyst compositions useful for the preparation of liquid hydrocarbons from synthesis gas. These catalyst compositions are characterized, in particular, as cobalt-titania or thoria promoted cobalt-titania, wherein cobalt, or cobalt and thoria, is composited or dispersed upon titania, or titania-containing support, especially a high rutile content titania. U.S. 4,568,663, also discloses cobalt-titania catalysts to which rhenium is added to improve catalyst activity, and regeneration stability. These catalysts have performed admirably well in conducting Fischer-Tropsch reactions, and in contrast to earlier cobalt catalysts provided high liquid hydrocarbon selectivities, with relatively low methane formation.

More recent publications also disclose cobalt catalysts as well as a process for the preparation of such catalysts by immersion of a porous carrier once or repetitively within a solution containing a cobalt compound. The cobalt is dispersed over the porous carrier to satisfy the relation $\Sigma V_p/\Sigma V_c \leqq 0.85$ and $\Sigma V_p/\Sigma V_c \leqq 0.55$, respectively, where $\Sigma V_c$ represents the total volume of the catalyst particles and $\Sigma V_p$ the peel volumes present in the catalyst particles, the catalyst particles being regarded as constituted of a kernal surrounded by a peel. The kernal is further defined as one of such shape that at every point of the kernal perimeter the shortest distance (d) to the perimeter of the peel is the same, d being equal for all particles under consideration, and having been chosen such that the quantity of cobalt present in $\Sigma V_p$ is 90% of the quantity of cobalt present in $\Sigma V_c$. These particular catalysts, it is disclosed, show higher $C_{5+}$ selectivities than catalysts otherwise similar except that the cobalt component thereof is homogeneously

2

distributed, or uniformly dispersed, throughout the carrier. Suitable porous carriers are disclosed as silica, alumina, or silica-alumina, and of these silica is preferred. Zirconium, titanium , chromium and ruthenium are disclosed as preferred of a broader group of promoters. Albeit these catalysts may provide better selectivities in synthesis gas conversion reactions via-a-vis catalysts otherwise similar except the cobalt is uniformly dispersed throughout the carrier, like other cobalt catalysts disclosed in the prior art, the intrinsic activities of these catalysts are too low as a consequence of which higher temperatures are required to obtain a productivity which is desirable for commercial operations. Higher temperature operation, however, leads to a corresponding increase in the methane selectivity and a decrease in the production of the more valuable liquid hydrocarbons.

Productivity, is defined as the standard volumes of carbon monoxide converted/volume catalyst/hour. High productivities are essential in achieving commercially viable operations. However, it is also essential that high productivity be achieved without high methane formation, for methane production results in lower production of liquid hydrocarbons. Accordingly, an important and necessary objective in the production and development of catalysts is to produce catalysts which are capable of high productivity, combined with low methane selectivity.

Despite improvements, there nontheless remains a need for catalysts capable of increased productivity, without increased methane selectivity. There is, in particular, a need to provide further improved catalysts, and process for the use of these catalysts in synthesis gas conversion reactions, to provide further increased liquid hydrocarbon selectivity, especially $C_{10+}$ liquid hydrocarbon selectivity, with further reduced methane formation.

It has been proposed that both needs may be met with available catalyst powder of the size 80-140 mesh (approximately 0.15 mm in diameter). However, additional factors should be considered in the design of a fixed bed reactor; namely, the pressure drop in the reactor, and the removal of the heat generated by the reaction.

These require the design of catalyst pellets which retain the properties of the powder catalyst (80-140 mesh) but are larger in size (> 1.0 mm). However, since the reactants have to diffuse through liquid-filled pores, the longer diffusion path may create concentration gradients within the pellet. Such gradients alter the hydrogen to carbon monoxide ratio in the pellet due to the lower diffusivity of the latter. As a result the selectivity to methane, which depends on this ratio, increases considerable. Furthermore, since the rate of reaction depends on the concentration of the two reactants, the productivity is smaller in a pellet than in powder.

Because the pellets have to be used in a fixed bed reactor, the design of the catalyst pellet has to be directed toward minimizing the methane selectivity and maximizing the productivity. The catalyst of the present invention is designed to achieve this purpose.

US-A-2,606,159 describes catalyst compositions for hydrocarbon conversion reactions in which the catalyst metal, preferably chromium but mentioning cobalt, is dispersed in an outer layer of a refractory oxide pellet which is rendered more porous than the interior by the exclusion of binder. Pellets particularly described are 1/8 by 1/8 inch (3 mm) cylindrical pellets having cores of 1/16-3/32 inch (1.59-2.38 mm) diameter, thereby leaving outer active layers containing the catalyst metal of 1/64-1/32 inch (0.39-0.79 mm).

According to the invention, there is provided a catalyst composition useful for the conversion of synthesis gas to hydrocarbons at high productivity, with low methane selectivity, comprising cobalt dispersed as a catalytically-active layer at the surface of a support, characterised in that said layer has a thickness in the range of from 0.02 to 0.25 mm. In a preferred embodiment, the thickness of the catalytically-active layer may be up to 0.18 mm, and one preferred range of thicknesses of the said layer may be from 0.04 to 0.15 mm.

The catalyst composition may have a loading of cobalt in the range of from 0.01 to 0.15 $g/cm^3$, calculated as metallic cobalt per packed bulk volume of catalyst.

The catalyst composition may be in the form of particles (e.g., beads, spheres, extrudates, rings, saddles) or pellets having a diameter in the range of from 0.5 to 2.0 mm.

In a preferred form of the invention, said layer thickness is a predetermined optimal layer thickness obtainable by calculation, for a said catalyst of productivity in excess of 150 $h^{-1}$, by relating predicted rates of diffusion of CO and $H_2$ to a predicted rate of reaction.

The cobalt may be present with or without the additional presence of other metals as promoters.

In a preferred embodiment of the invention the catalyst of the present invention may comprise a particulate cobalt catalyst formed by dispersing cobalt as a thin catalytically active film upon the surface of a particulate support, preferably a titania - containing support, especially one wherein the rutile: anatase ratio of the titania support is at least about 3:2.

3

The catalysts of this invention can be used to produce, by contact and reaction at reaction conditions with an admixture of carbon monoxide and hydrogen, a distillate fuel constituted principally of an admixture of linear paraffins and olefins, particularly a $C_{10+}$ distillate, at high productivity, with low methane selectively. This product can be further refined and upgraded to high quality fuels, and other products such as mogas, diesel fuel and jet fuel, especially premium middle distillate fuels of carbon numbers ranging from about $C_{10}$ to about $C_{20}$.

As stated above, the design of the preferred catalyst requires a consideration of the rate of diffusion of the CO and the $H_2$ and relating these rates to a rate of reaction in the porous inorganic oxide for a predetermined support geometry, partial pressures and temperatures.

The concentrations of both hydrogen and carbon monoxide decrease as they diffuse into the catalyst pellet due to significant mass transfer resistance inside the pores. The global rate of CO conversion in the pellet decreases. Furthermore, the methane production rate increases which is a result of its dependency on the ratio between the hydrogen to CO concentration which increases in the pellet. This ratio increases if the parameter r (see equation 11 below) is less than unity. This behavior was observed in Co or Ru catalysts supported on titania, silica or alumina.

At a certain depth in the pellet, the hydrogen to CO ratio reaches values which cause most of the CO to be converted to methane which is detrimental to the process. This depth which is called the optimum rim thickness can be determined from the pellet model. Increasing the rim thickness diverts most of the marginal CO conversion to methane while decreasing the rim thickness decreases the CO conversion significantly. Therefore, for an optimal operation, the optimal rim thickness should be determined.

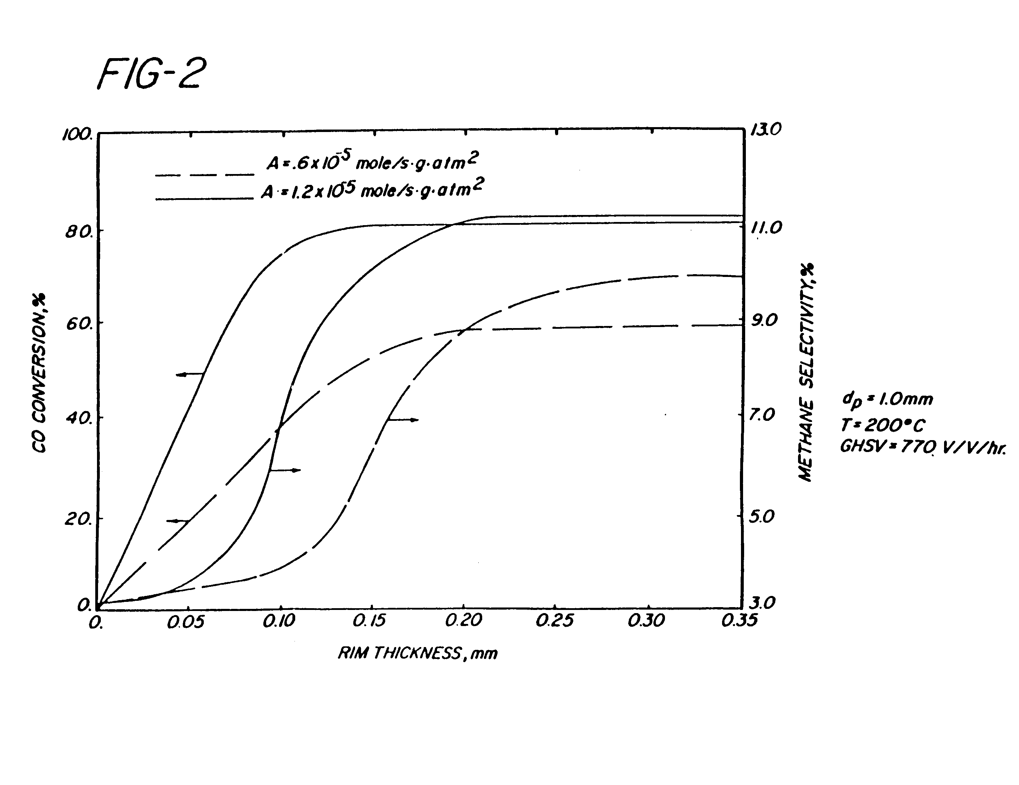

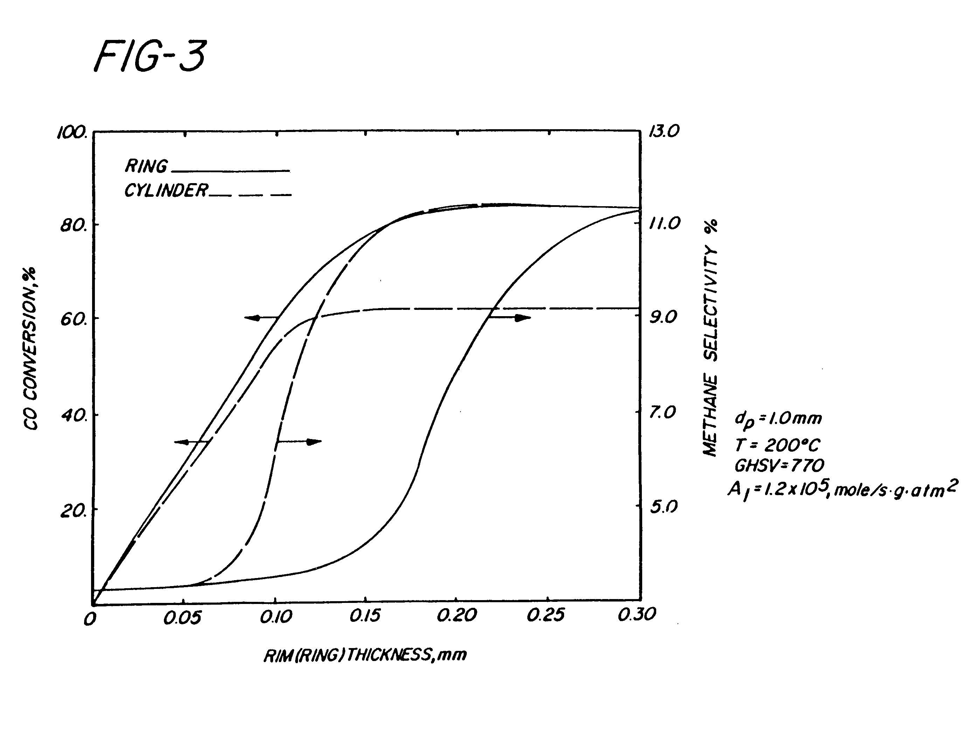

As will be discussed below with respect to Figures 2 and 3, it is not possible to simultaneously maximize CO conversion and minimize methane conversion. However, it is possible to choose a rim thickness so as to optimize CO conversion to heavy hydrocarbons.

The process of the present invention is carried out at a rate of CO conversion to hydrocarbons such that the percentage of methane production is maintained at a predetermined low level so as to make the entire process useful and practical.

As the rim thickness increases above the optimal rim thickness, the marginal increase in CO conversion is accompanied by an increase in the percentage of methane production in the converted carbon monoxide. This is observed in Figure 2 and 3 discussed below. At the optimal rim thickness, most of the increase in CO conversion goes into methane production. Since an object of the present invention is to limit methane production in the converted CO, a rim thickness must be chosen at about this value.

The supported catalyst may be fabricated by a number of different methods known in the art, see e.g., Scientific Basis For The preparation of Heterogenous Catalyst, Preprints of the Fourth International Symposium, September 1-4, 1986, Louvain-La-Nueve, Belgium.

In the drawings:-

Figure 1 shows the model predictions for methane selectivity as a function of pellet radius compared with experimental data.

Figure 2 shows carbon monoxide conversion and methane selectivity as a function of rim thickness for spherical pellets.

Figure 3 shows carbon monoxide conversion and methane selectivity as a function of rim thickness for a ring, and cylindrical pellets.

Figure 4 is a graph summarizing the methane selectivity produced at a given productivity for each of 21 catalysts described in tables 4-6.

## CATALYST DESIGN CONSIDERATIONS

The two reactants, hydrogen and carbon monoxide, diffuse in the liquid filled pores to reach the active metal sites on the support. The function of the support is to increase the surface area which is equal to about 20 $m^2/g$ in this case. At steady-state, the fluxes of the two reactants in the pores are equal (since there is no accumulation):

$$\beta D_{e,CO} \frac{d C_{CO}}{d x} = D_{e,H} \frac{d C_H}{d x} \qquad (1)$$

where the flux is expressed as a product of the effective diffusivity $D_e$ and the concentration gradient. $\beta$ is the stoichiometric coefficient which is equal to 2.07 for the hydrocarbon synthesis reaction. The ratio of the gradients depends on the ratio of the two diffusivities. Since the diffusivity of hydrogen is greater than that of carbon monoxide, the hydrogen to carbon monoxide ratio is expected to increase, moving from the pellet surface towards its center.

A differential mass balance inside the pellet pores for the carbon monoxide (which is the limiting reactant, namely it is depleted before the other reactant) yields:

$$D_{e,co}\ \frac{1}{x^s}\frac{d}{dx}\left(x^s\ \frac{dC_{co}}{dx}\right) = \rho_p r_{co} \qquad (2)$$

where x is the radial position measured from the external surface toward the center, $\rho p$ is the pellet density $C_{co}$ is the CO concentration in the liquid-filled pores and $r_{co}$ is the intrinsic rate of reaction on the active sites. s is equal to two for a sphere and to unity for a cylinder. This analysis has been carried out for a sphere or a cylinder. However, the analysis can be extended to three shapes. For example, rims having a ring shape or a semi-circular shape may be used.

The boundary condition on the external surface is:

$$C_{co} = P_{co,b}/H_{co} \quad x = x_s \qquad (3)$$

where $P_{co,b}$ is the CO partial pressure in the bulk gas phase and $H_{co}$ is the Henry's law constant. The other boundary condition is set in two cases:

1. inert core

$$\frac{dC_{co}}{dx} = 0 \quad x = x_i \qquad (4)$$

2. hollow core

$$C_{co} = P_{co,b}/H_{co} \quad x = x_i \qquad (5)$$

In the derivation of the boundary conditions it is assumed that the external mass transfer resistance is negligible. This assumption was tested both experimentally and theoretically. Furthermore, the pellet is assumed to be isothermal, based on calculations which indicated temperature gradients less than 0.1 degree C, as expected for liquid-filled porous catalysts.

Equation (2) is general for any reaction with diffusion, while the rate of reaction depends on the catalyst system. The intrinsic rate expression (free of internal or external mass transfer resistance) for catalyst systems such as cobalt or ruthenium or titania or silica can be written as:

$$r_{co} = k_1\ exp\left(-\frac{E_1}{RT}\right)\frac{P_{co}^a\ P_H^b}{(1 + k_2 P_{co} + k_3 P_H)^2} \qquad (6)$$

The values of $k_1$, $k_2$, $k_3$, $E_1$, a, b and c are calculated from kinetic rate data obtained in laboratory reactors. The kinetic parameter $k_1$ usually depends only on the metal concentration on the support. However, in certain cases such as cobalt on titania, it is also a function of the water partial pressure:

5

$$k_1 = A \ \frac{1 + k_4 \, P_{H_2O}}{1 + (k_5 \, P_{H_2O})^2} \qquad (7)$$

where A is the activity of the catalyst.

Equation (6) can be expressed in terms of the CO and $H_2$ concentrations using the Henry's law:

$$C_{co} = P_{co}/H_{co} \ ; \ C_H = P_H/H_H \qquad (8)$$

Furthermore, the $H_2$ concentration can be expressed in terms of the CO concentration by integrating equation (1) to give

$$C_H = \frac{P_H \, b}{H_H} - \frac{\beta D_{e,co}}{D_{e,H}}\left(\frac{P_{co,b}}{H_{co}} - C_{co}\right) \qquad (9)$$

or

$$\frac{H_H C_H}{P_{H,b}} = 1 - \gamma\left(1 - \frac{H_{co} \, C_{co}}{P_{co,b}}\right) \qquad (10)$$

where

$$\gamma = \frac{\beta D_{e,co}}{D_{e,H}} \ \frac{H_H}{H_{co}} \ \frac{P_{co,b}}{P_{H,b}} \qquad (11)$$

Substituting equations (6) and (10) into equation (2) and expressing the equation in dimensionless form yields the dimensionless number

$$\phi = (x_s - x_c)\left[\frac{\rho_P \, k_1 \, exp(-\frac{E_1}{RT}) \, H_{co} \, P_{H,b}}{D_{e,co} \, (k_2 \, P_{co,b})^e}\right]^{\frac{1}{2}} \qquad (12)$$

$\phi$, called the Thiele modulus, is the ratio between the maximum rate of reaction and the maximum rate of diffusion. If $\phi \gg 1$ the process is diffusion limited while for $\phi \ll 1$ the process is kinetic limited. Since $\phi$ is directly proportional to the thickness of the active layer or rim, diffusion is important in pellets and negligible in powder. The other factors affecting $\phi$ are the partial pressures, temperatures and the catalyst activity (metal loading).

r expresses the ratio between the maximum rate of diffusion of the two reactants. If r = 1, the ratio of carbon monoxide to hydrogen remains unchanged in the pores while for r < 1 this ratio decreases.

Equation (2) is solved to yield the concentration profiles in the pores of the pellet. Then the concentration profiles are integrated over the volume of the pellet to calculate the effectiveness factor which is the ratio of the actual rate of reaction (called the global rate of reaction) and the maximum rate reaction calculated at the surface conditions:

$$\eta_{co} = \frac{\frac{1}{V}\int_{v_p} r_{co}\, dV}{r_{co}\,(P_H,\, P_{co},\, P_{H_2O})} \qquad (13)$$

The same concentration profiles are integrated using the rate of methane production rate $r_{CH_4}$ to yield the effectiveness factor for methane

$$\eta_{CH_4} = \frac{\frac{1}{V_p}\int_{v_p} r_{CH_4}\, dV}{r_{CH_4}\,(P_H,\, P_{co},\, P_{H_2O})} \qquad (14)$$

$r_{CH_4}$ was also obtained from kinetic measurements:

$$r_{CH_4} = k_4\, exp\left(\frac{-(E_2-E_1)}{RT}\right)\frac{P_H}{1+k_2\,P_{co}+k_3\,P_H}\, r_{co} \qquad (15)$$

Finally, $\eta_{co}$ and $\eta_{CH_4}$ are employed in the reactor mass balance to calculate the carbon monoxide conversion and the methane selectivity. For simplicity, the reactor is assumed to be isothermal:

$$Y_{co,i}\, \frac{G_f}{M_i}\frac{dx_{co}}{dZ} = \eta_{co}\, \rho_B\, r_{co} \qquad (16)$$

$$Y_{co,i}\, \frac{G_f}{M_i}\frac{dx_{CH_4}}{dZ} = \eta_{CH_4}\, \rho_B\, r_{CH_4} \qquad (17)$$

$$Z=0 \quad X_{CH_4}=0 \quad X_{co}=0$$

where $Y_{co,i}$ is the carbon monoxide mole fraction in the feed, $G_f$ is the mass velocity, $M_i$ is the molecular weight of the feed, $\rho_B$ is the bed density and $x_{co}$ and $x_{CH_4}$ are the carbon monoxide and methane conversion, respectively.

Although the fixed bed reactor is nonisothermal, the results presented here hold also in this case. Since the temperature increase never exceeds 30 degrees F (16.7°C), the optimal rim thickness can be calculated at the average temperature in the bed.

Estimation of Effective Diffusivities

The carbon monoxide conversion and the methane selectivity were measured with a 6% Co-0.5% Re catalyst of different pellet sizes. The hydrogen to CO ratio in the feed was 2.0. Those data were used to estimate the carbon monoxide and hydrogen effective diffusivities following the procedure:

Values of $D_{e,C}$ and $D_{e,H}$ were assumed;

The effectiveness factors $\eta_{CO}$ and $\eta_{CH_4}$ were calculated from equations (9) and (10) and the solution of equation (2) given the inlet conditions to the reactor;

Then the carbon monoxide conversion and the conversion to methane were calculated by integrating equations (16) and (17). Since the effectiveness factors are a function of the carbon monoxide, hydrogen and water partial pressures, the effectiveness factors were recalculated along the reactor as the partial pressures changed;

The methane selectivity was calculated from the ratio of the conversion to methane $X_{CH_4}$ and the carbon monoxide conversion $X_{CO}$;

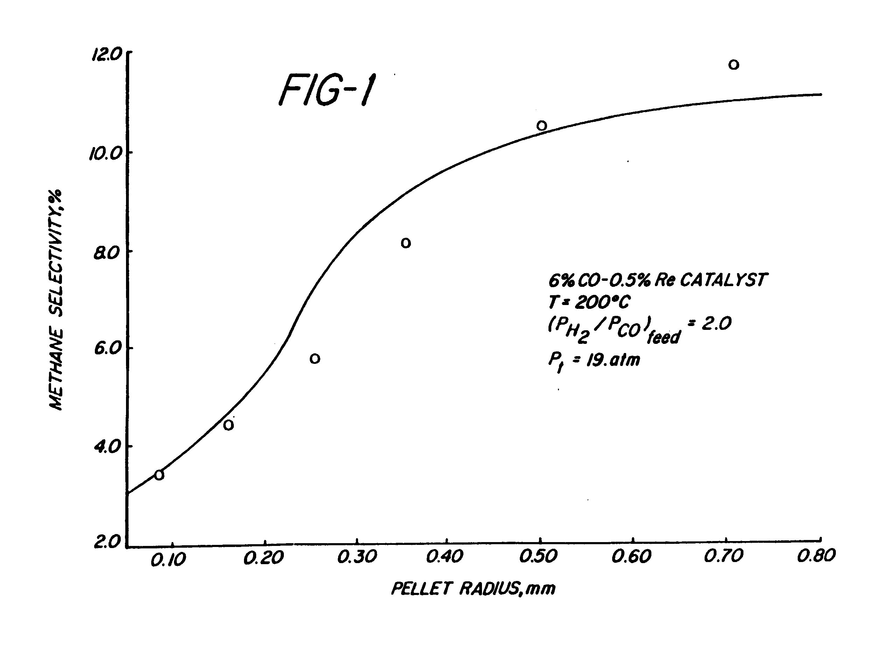

The calculated carbon monoxide conversion and the methane selectivity were compared with the experimental values for the various pellet sizes;
and
The effective diffusivities are adjusted to give the best fit of the experimental data. A comparison between the predictions and the data are given in Figure 1 and Table 1.

PREFERRED CATALYSTS

In the preferred catalyst compositions the catalytically active metallic component is dispersed and supported upon a refractory inorganic oxide carrier or support in the form of particles or pellets, generally hereafter called particles, as a thin catalytically active surface layer, film, or rim whose optimum thickness may be determined by relating the rate of diffusion of the CO and hydrogen in the feed to the reaction zone to a rate of reaction in the refractory inorganic oxide carrier or support for a predetermined support geometry, partial pressures and temperature. Suitable supports are, e.g. silica, silica-alumina, alumina or titania. Titania or a titania-containing support is preferred, especially a titania wherein the rutile:anatase ratio is at least about 3:2. The feature of a high metal loading in a thin catalytically active layer located at the surface of the particles, while the metal is substantially excluded from the inner surface of the particles, is essential in optimizing the activity, selectivity and productivity of the catalyst in producing liquid hydrocarbons from synthesis gas, while minimizing methane formation.

Metals such as rhenium, zirconium, hafnium, cerium, thorium and uranium or the compounds thereof, can be added to the active metal catalyst, i.e. cobalt, to increase the activity and regenerability of the catalyst. Thus, the thin catalytically active layers, or films, formed on the surface of the support particles, especially the titania or titania containing support particles, can include in addition to a catalytically active amount of cobalt, any one or more of rhenium, zirconium, hafnium, cerium, uranium, and thorium, or admixtures of these with each other or with other metals or compounds thereof.

When cobalt is the active metallic component dispersed and supported upon a particulate refractory inorganic oxide carrier or support as a thin catalytically active surface layer, film or rim the thickness of this layer, film or rim will range from 0.02 millimeters (mm) to 0.25 mm, e.g., up to 0.18 mm, preferably from 0.04 mm to 0.15 mm. Preferably, the loading of the cobalt expressed as the weight metallic cobalt per packed bulk volume of catalyst will be in the range of from 0.01 grams (g) per cubic centimeter ($cm^3$) to 0.15 $g/cm^3$, more preferably from 0.03 $g/cm^3$ to 0.09 $g/cm^3$ catalyst. Preferred thin catalytically-active layers, or films, supported on a support, notably a titania or a titania-containing support, thus include cobalt-rhenium, cobalt-zirconium, cobalt-hafnium, cobalt-cerium, cobalt-uranium, and cobalt-thorium, with or without the additional presence of other metals or compounds thereof.

A particularly preferred catalyst is one wherein the cobalt, or the cobalt and a promoter, is dispersed as a thin catalytically active film upon titania, $TiO_2$, or a titania-containing carrier, or support, in which the titania has a rutile:anatase weight ratio of at least about 3:2, as determined by ASTM D 3720-78: Standard Test Method for Ratio of Anatase to Rutile In Titanium Dioxide Pigments By Use of X-Ray Diffraction. Generally, the catalyst support may be one wherein the titania has a rutile:anatase ratio in the range of at least 3:2 to 100:1, or greater, and more preferably from 4:1 to 100:1, or greater. Where any one of rhenium, zirconium, hafnium, cerium, thorium, or uranium metals, respectively, is added to the cobalt as a promoter to form the thin catalytically active film, the metal is added to the cobalt in concentration sufficient to provide a weight ratio of cobalt:metal promoter ranging from 30:1 to 2:1, preferably from 20:1 to 5:1. Rhenium and hafnium are the preferred promoter metals, rhenium being more effective in promoting improved activity maintenance on an absolute basis, with hafnium being more effective on a cost-effectiveness basis. These catalyst compositions, it has been found, produce at high productivity, with low methane selectivity, a product which is predominately $C_{10+}$ linear paraffins and olefins, with very little oxygenates. These catalysts also provide high activity, high selectivity and high activity maintenance in the conversion of carbon monoxide and hydrogen to distillate fuels.

The cobalt catalysts of this invention, as a contrasted with (i) cobalt catalyst, the cobalt portion of which is uniformly distributed throughout the support particles or (ii) cobalt catalysts having a relatively thick surface layer of cobalt on the support particles, have proven especially useful for the preparation of liquid hydrocarbons from synthesis gas at high productivities, with low methane formation. In contrast with the catalysts of this invention, the prior art catalysts are found to have lower activity, and especially poorer selectivity due to sever diffusion limitations. These catalysts (i) and (ii), supra, at high productivities, produce altogether too much methane. As productivity is increased to produce greater conversion of the carbon monoxide to hydrocarbons, increased amounts of methane are concurrently produced. It was thus found that increased productivity with these catalysts could only be obtained at the cost of increased

8

methane formation. This result occurs, it is believed, because the carbon monoxide and hydrogen reactants all too slowly diffuse through the pores of the particulate catalyst which becomes filled with a liquid product, this resulting in underutilization of the catalytically active sites located within the interior of the particles. Both hydrogen and carbon monoxide must thus diffuse through the product-liquid filled pores, but hydrogen diffuses through the pores at a greater rate of speed than the carbon monoxide. Since both the hydrogen and the carbon monoxide are reacting at the catalytic sites at an equivalent rate, a high $H_2/CO$ ratio is created in the interior of the particle which leads to high methane formation. As the rate of reaction is increased, e.g., by incorporating higher intrinsic activity or by operating at higher temperature, the catalyst becomes more limited by the rate of diffusion of the reactants through the pores. Selectivities are especially poor under the conditions of high productivity. Thus, the catalyst used during a Fischer-Tropsch hydrocarbon synthesis reaction is one the pores of which become filled with the product liquid. When the CO and $H_2$ are passed over the bed of catalyst and consumed at a rate which is faster then the rate of diffusion, $H_2$ progresses to interior of the particle to a much greater extent than the CO, leaving the interior of the particles rich in $H_2$, and deficient in CO. The formation of methane within the particle interior is thus favored due to the abnormally high $H_2/CO$ ratio; an unfavorable result since $CH_4$ is not a desirable product. The extent to which selectivity is debited depends on the magnitude of the difference between the rate of diffusion and the rate of reaction, i.e., the productivity.

The catalyst of this invention is thus one wherein essentially all of the active cobalt is deposited on the surface of the support particles, preferably titania or titania-containing support particles, while cobalt is substantially excluded from the inner surface of the particles. The surface film of cobalt must be very thin and contain an adequate loading of cobalt to maximize reaction of the hydrogen and carbon monoxide at the surface of the catalytic particle. The promoter metal to be effective must also be contained within the surface film of cobalt. If extended into the interior of the particle outside the cobalt film the promoter metal will have little promotional effect, if any. The metal promoter should thus also be concentrated within the cobalt film at the surface of the catalyst, with the weight ratio of cobalt:metal promoter, as suggested, ranging from 30:1 to 2:1, preferably from 20:1 to 5:1. The thickness of the surface metal film can be conveniently measured by an Electron Probe Analyzer, e.g., one such as produced by the JEOL Company, Model No. JXA-50A. Cross sections of the catalyst particles of this invention measured via use of this instrument show very high peaks, or shoulders, at the edges of the particle across the particle across the line of sweep representative of cobalt concentration, with little or no cobalt showing within the particle interior. The edge, or "rim" of the "radially impregnated catalyst" will thus contain essentially all of the cobalt added to the catalyst, The thickness of the film, or rim, is unrelated to the absolute size, or shape of the support particles. Virtually any size particle can be employed as is normally employed to effect catalyst reactions of this type, the diameter of the particle ranging generally from about 0.5 mm to about 2 mm. The particles can be of virtually any shape, e.g., as is normally employed to effect reactions of this type, viz., as beads or spheres, extrudates, ring, saddles or the like. By concentrating the catalytic metal, or metals, on the extreme outer surface of the particles, the normal diffusion limitation of the catalyst can be overcome. This new catalyst is more active in its function of bringing about a reaction between the CO and $H_2$. The catalyst because of its having the thin layer of catalytically active metal on its surface is in effect found to behave more ideally, approaching, in fact, the behavior of a powdered catalyst which is not diffusion limited. However, unlike in the use of powdered catalysts the flow of the reactants through the catalyst bed, because of the larger particle size of the catalyst, is virtually unimpeded. Higher productivity, with lower methane selectivity, is the result; a result of considerable commercial consequence. At productivities greater than 150 $hr^{-1}$ (standard volumes of carbon monoxide converted per volume of catalyst per hour), notably from 150 $hr^{-1}$ to 200 $hr^{-1}$, less than 10 mole percent of the carbon monoxide converted is converted to methane.

In conducting synthesis gas reactions the total pressure upon the CO and $H_2$ reaction mixture is generally maintained above about 80 psig (551.6 kPa), and preferably above about 140 psig (965.3 kPa). It is generally desirable to employ carbon monoxide, and hydrogen, in molar ratio of $H_2:CO$ above about 0.5:1 and preferably equal to or above about 1.7:1 to increase the concentration of $C_{10+}$ hydrocarbons in the product. Suitably, the $H_2:CO$ molar ratio ranges from 0.5:1 to 4:1, and preferably the carbon monoxide and hydrogen are employed in molar ratio $H_2:CO$ ranging from 1.7:1 to 2.5:1. In general, the reaction is carried out at gas hourly space velocities ranging from 100 V/Hr/V to 5000 V/Hr/V, preferably from 300 V/Hr/V to 1500 V/Hr/V, measured as standard volumes of the gaseous mixture of carbon monoxide and hydrogen (0 degrees C, 1 Atm.) per hour per volume of catalyst. The reaction is conducted at temperatures ranging from about 160 degrees C to about 290 degrees C, preferably from about 190 degrees C to about 260 degrees C. Pressures preferably range from about 551 kPa (80 psig) to about 4134 kPa (600 psig), more preferably from about 965 kPa (140 psig) to about 2756 kPa (400 psig). The product generally and preferably contains

9

60 percent, or greater, and more preferably 75 percent, or greater, $C_{10+}$ liquid hydrocarbons which boil above 160 degrees C (320 degrees F).

The catalysts employed in the practice of this invention can be prepared by spray techniques where a dilute solution of a cobalt compound, alone or in admixture with a promoter metal compound, or compounds, as a spray is repetitively contacted with the hot support particles, e.g., the titania, or titania-containing support particles. The particulate support, e.g., the titania or titania-containing support particles, are maintained at temperatures equal to or above about 140 degrees C when contacted with the spray, and suitably the temperature of the support, e.g., the titania, or titania-containing support, ranges from about 140 degrees C up to the decomposition temperature of the cobalt compound, or compounds in admixture therewith; preferably from about 140 degrees C to about 190 degrees C. The cobalt compound employed in the solution can be any organometallic or inorganic compound which decomposes to give cobalt oxide upon initial contact or upon calcination, such as cobalt nitrate, cobalt acetate, cobalt acetylacetonate, cobalt naphthenate, cobalt carbonyl, or the like. Cobalt nitrate is especially preferred while cobalt halide and sulfate salts should generally be avoided. The cobalt salts may be dissolved in a suitable solvent, e.g., water, organic or hydrocarbon solvent such as acetone, methanol, pentane or the like. The total amount of solution used should be sufficient to supply the proper catalyst loading, with the film being build up by repetitive contacts between the support and the solvent. The preferred catalyst is one which consists essentially of cobalt, or cobalt and promoter, dispersed upon the titania, or titania-containing support, support, especially a rutile support. Suitably, the hot support, notably the titania support, is contacted with a spray which contains from about 0.05 g of cobalt/ml of solution to about 0.25 g of cobalt/ml of solution, preferably from about 0.10 g of cobalt/ml of solution to about 0.20 g of cobalt/ml of solution, (plus the compound containing the promoter metal, if desired), generally from at least about 3 to about 12 contacts, preferably from about 5 to about 8 contacts, with intervening drying and calcination steps being required to form surface films of the required thickness. The hot support, titania, or titania- containing support, in other words, is spray-contacted in a first cycle which includes the spray contact per se with subsequent drying and calcination, a second cycle which includes the spray contact per se with subsequent drying and calcination, a third spray contact which includes the spray contact per se with subsequent drying and calcination, etc. to form a film of the required thickness and composition. The drying steps are generally conducted at temperatures ranging above about 20 degrees C, preferably from about 20 degrees C to about 125 degrees C, and the calcination steps at temperatures ranging above about 150 degrees C, preferably from about 150 degrees C to about 300 degrees C.

Titania is the preferred support. It is used as a support, either along or in combination with other materials for forming a support. The titania used for the support is preferably one which contains a rutile:anatase ratio of at least about 3:2, as determined by x-ray diffraction (ASTM D 3720-78). The titania supports preferably contain a rutile:anatase ratio of from about 3:2 to about 100:1, or greater, more preferably from about 4:1 to about 100:1, or greater. The surface area of such forms of titania are less than about 50 $m^2/g$. These weight concentrations of rutile provide generally optimum activity, and $C_{10+}$ hydrocarbon selectivity without significant gas and $CO_2$ make.

The prepared catalyst as a final step is dried by heating at a temperature above about 20 degrees C, preferably between 20 degrees C and 125 degrees C, in the presence of nitrogen or oxygen, or both, in an air stream or under vacuum. It is necessary to activate the catalyst prior to use. Preferably, the catalyst is contacted with oxygen, air, or other oxygen-containing gas at temperature sufficient to oxidize the cobalt and convert the cobalt to $Co_3O_4$. Temperatures ranging above about 150 degrees C, and preferably above about 200 degrees C are satisfactory to convert the cobalt to the oxide, but temperatures above about 500 degrees C are to be avoided unless necessary for regeneration of a severely deactivated catalyst. Suitably, the oxidation of the cobalt is achieved at temperatures ranging from about 150 degrees C to about 300 degrees C. The metal, or metals, contained on the catalyst are then reduced. Reduction is performed by contact of the catalyst, whether or not previously oxidized, with a reducing gas, suitably with hydrogen or a hydrogen-containing gas stream at temperatures above about 200 degrees C; preferably above about 250 degrees C. Suitably, the catalyst is reduced at temperatures ranging from about 200 degrees C to about 500 degrees C for periods ranging from about 0.5 to about 24 hours at pressures ranging from ambient to about 40 atmospheres. A gas containing hydrogen and inert components in admixture is satisfactory for use in carrying out the reduction.

The catalysts of this invention can be regenerated, and reactivated to restore their initial activity and selectivity after use by washing the catalyst with a hydrocarbon solvent, or by stripping with a gas. Preferably the catalyst is stripped with a gas, most preferably with hydrogen, or a gas which is inert or nonreactive at stripping conditions such as nitrogen, carbon dioxide, or methane. The stripping removes the hydrocarbons which are liquid at reaction conditions. Gas stripping can be performed at substantially the same temperatures and pressures at which the reaction is carried out. Pressures can be lower however, as low as atmospheric or even a vacuum. Temperatures can thus range from about 160 degrees C to about 290 degrees C, preferably from about 190 degrees C to about 260 degrees C, and pressures from below atmospheric to about 4314 kPa (600 psig). If it is necessary to remove coke from the catalyst, the catalyst can be contacted with a dilute oxygen-containing gas and the coke burned from the catalyst at controlled temperature below the sintering temperature of the catalyst. Most of the coke can be readily removed in this way. The catalyst is then reactivated, reduced, and made ready for use by treatment with hydrogen or hydrogen-containing gas with a fresh catalyst.

The invention will be more fully understood by reference to the following examples and demonstrations which present comparative data illustrating its more salient features.

Example 1

Simulations of the carbon monoxide conversion and methane selectivity as a function of the rim thickness are depicted in Figure 2 for a spherical pellet loaded with catalysts having two different activities. A hydrogen to CO ratio of 2.0 in the feed was assumed. The carbon monoxide conversion increases with with the rim thickness up to its maximum value. The methane selectivity remains almost constant up to certain rim thickness where it increases steeply. Over a range of rim thicknesses the carbon monoxide conversion reacts almost its maximum value while the methane selectivity is still low. Specifically, in Figure 2 for a spherical pellet with an activity of $6 \times 10^2$ mol/s/g/kPa$^2$ ($6 \times 10^4$ mol/s/g/atm$^2$) the range is 0.13 to 0.15 mm while for an activity of $1 \times 10^2$ mol/s/g/kPa$^2$ ($1.2 \times 10^5$ mol/s/g/atm$^2$) the range is 0.08 to 0.10 mm. This illustrates the essence of the invention. Based on design specification, a rim thickness can be determined to give both a high carbon monoxide conversion and a low methane selectivity. The exact value of the rim thickness depends on the catalyst activity, the partial pressures, the temperature and the pellet shape and configuration. An example of a ring and a cylinder is given in Figure 3. The behavior of those pellet is similar in terms of the effect of rim thickness on CO conversion and methane selectivity. The detailed calculations are carried out for each pellet shape using the general reaction and diffusion model.

Example 2

Experiments were performed in a reactor 91 cm (3 ft.) long and 1.3 cm (0.5 in.) in diameter. The reactor was packed with 1 mm dia. spherical catalyst pellets loaded uniformly with 6% Co - 0.5% Re. Isothermal conditions were maintained in the reactor.

Data obtained at various temperatures and hydrogen to CO ratios in the feed were compared with the predictions of the model. As explained previously, the carbon monoxide conversion and methane selectivity were obtained by integrating equations (16) and (17) and calculating the effectiveness factors from equations (13) and (14).

In the experiments with a hydrogen to CO ratio in the feed of less than the stoichiometric ratio (2.07), the ratio decreased along the bed. Since the methane selectivity depends on this ratio, it decreased as the hydrogen to CO decreased. Furthermore, since the diffusion in the catalyst pores was one of the limiting steps in this system, a lower hydrogen to CO ratio increases the r parameter which means a lower methane selectivity. A comparison of experiments 4, 5 and 6 illustrates the improvements in methane selectivity. In experiment 3, the methane selectivity increased as compared with experiment 6 because the temperature was higher. The agreement for both the methane selectivity and the carbon monoxide conversion were good, as shown in Table 2.

EXAMPLE 3

Experiments were performed in a reactor 91 cm (3 feet) long and 1.3 cm (0.5 inches) in diameter. In this case the reactor was operated under nonisothermal conditions, namely the temperature changed along the reactor. The reactor was packed with a spherical rim pellet with 6% Co-.5% Re catalysts (based on the rim mass). The pellet size, rim thickness and catalyst activity are given in Table 3. The pressure was 20 atm.

The prediction of the carbon monoxide conversion and methane selectivity requires the solution of a heat balance for the reactor along with the mass balances in equations (16) and (17). A comparison of the temperature profiles, CO conversion and methane selectivity are given in Table 3.

The data indicate that the CO conversion is close to the maximum conversion attainable for those pellets under the given conditions (73% and 78% for Experiments A and B, respectively). However, the methane selectivity was lower than the 11% expected for those pellets as reported in Figure 1.

The rim tested in this Example was not of optimal size. A 0.1 mm rim would have lowered the methane selectivity to about 5% while keeping the conversion at about 70%.

EXAMPLE 4

Simulations were performed for a pellet of ring shape. The operating conditions assumed in the simulations are:

reactor diameter - 3.8 cm (1.5")
pellet outer diameter - 1.5 mm
pellet inner diameter - 1.0 mm
gas mass velocity - 0.39 kg/cm$^2$/h (800 lb/ft$^2$/h)
coolant temperature - 175°C (347 degrees F)
catalyst activity - 138 gmol/s/g/kPa$^2$ (1.4 x 10$^5$ gmol/s/g/atm$^2$)
feed composition - 64% H$_2$, 32% CO, and 4% N$_2$

The heat balance and the mass balance in equations (16) and (17) were solved to yield the following results:

maximum temperature rise - 30 degrees F (16.7°C)
CO conversion - 70%
methane selectivity - 5.6%

TABLE 1

| MODEL PREDICTIONS COMPARED WITH DATA | | | | |
|---|---|---|---|---|
| Pellet Radius mm | GHSV* | Bulk Density g/cm$^3$ | CO Conversion | |
| | | | exp. | pred. |
| 0.088 | 1500 | 1.47 | 76 | 74 |
| 0.166 | 1320 | 1.47 | 76 | 77 |
| 0.252 | 1000 | 1.53 | 78 | 83 |
| 0.356 | 1140 | 1.59 | 73 | 69 |
| 0.500 | 840 | 1.73 | 75 | 76 |
| 0.705 | 510 | 1.64 | 79 | 81 |

* Gas hourly space velocity.

EP 0 266 898 B1

## TABLE 2

### PREDICTED AND EXPERIMENTAL SELECTIVITIES
### AND CONVERSIONS

| Exp. No. | Temp., $^{o}C$ | Feed $H_2/CO$ | Selectivity, % exp. | pred. | CO Conversion, % exp. | pred. |
|---|---|---|---|---|---|---|
| 1 | 185 | 1.70 | 6.6 | 4.2 | 48 | 52 |
| 4 | 192 | 2.00 | 9.6 | 8.1 | 76 | 76 |
| 2 | 199 | 1.55 | 6.6 | 5.0 | 56 | 57 |
| 3 | 204 | 1.69 | 8.1 | 7.7 | 63 | 65 |
| 4 | 193 | 2.19 | 10.2 | 10.0 | 74 | 74 |
| 5 | 193 | 1.90 | 7.6 | 7.3 | 74 | 76 |
| 6* | 193 | 1.70 | 5.3 | 4.7 | 74 | 76 |

* The activity decreased by about 10%.

13

## Table 3

### MODEL PREDICTIONS AGREE WITH NON-ISOTHERMAL PELLET DATA

$D_p$ = 1.10 mm          Rim Thick. = 0.15 mm

$H_2/CO$ in the feed = 2.0

$k_1$ = 118 gmol/g/s/kPa$^2$
(12.0 x 10$^4$ gmol/g/s/atm$^2$)

| | EXPERIMENT A | | EXPERIMENT B | |
|---|---|---|---|---|
| | Exp. | Pred. | Exp. | Pred. |
| Co Conv., mol% | 70.6 | 71.6 | 75.3 | 76.6 |
| CH$_4$ Sel., mol% | 8.5 | 8.4 | 8.5 | 8.9 |

AXIAL POSITION          TEMPERATURE, ($^o$F) $^o$C

| | TH | TH | TH | TH |
|---|---|---|---|---|
| 0.0 | 191.7 (377) | | 198.3 (389) | |
| 0.09 | 195.0 (383) | 196.1 (385) | 203.3 (398) | 205.0 (401) |
| 0.19 | 196.1 (385) | 197.2 (387) | 205.0 (401) | 206.1 (403) |
| 0.29 | 197.8 (388) | 197.8 (388) | 207.2 (405) | 207.2 (405) |
| 0.39 | 198.9 (390) | 198.9 (390) | 207.8 (406) | 207.8 (406) |
| 0.49 | 198.9 (390) | 198.9 (390) | 207.2 (405) | 207.2 (405) |
| 0.59 | 198.9 (390) | 198.9 (390) | 207.2 (405) | 206.7 (404) |
| 0.69 | 197.8 (388) | 198.3 (389) | 205.6 (402) | 205.6 (402) |
| 0.79 | 197.2 (387) | 197.8 (388) | 204.4 (400) | 203.9 (399) |
| 0.89 | 196.1 (385) | 197.2 (387) | 202.8 (397) | 202.8 (397) |
| 0.99 | 193.9 (381) | 196.1 (385) | 200.6 (393) | 201.7 (395) |

The catalysts of this invention are disclosed in the following examples and demonstrations as Catalysts Nos. 14-21. These are catalysts which have surface film falling within the required range of thicknesses, and the surface film contains the required cobalt metal loadings. It will be observed that all of Catalysts No. 14-21 were formed by a process wherein a heated particulate $TiO_2$ substrate was repetitively contacted with a dilute spray solution containing both the cobalt and rhenium which was deposited as a thin surface layer, or film, upon the particles. Catalysts No.s 14-21 are contrasted in a series of synthesis gas conversion runs with Catalysts Nos. 1-8, catalysts wherein the metals are uniformly dispersed throughout the $TiO_2$ support particles. They are also contrasted in runs made with Catalysts Nos. 9-13, also "rim" catalysts but catalysts wherein the surface films, or rims, are too thick (Catalysts Nos. 10-13), however prepared, or do not contain an adequate cobalt metal loading within the surface film, or rim (Catalyst No. 9). As will be observed from the data presented, it is clear that at high productivities the catalysts formed from the uniformly impregnated $TiO_2$ spheres produce high methane. Moreover, even wherein a film of the catalytic metal is formed on the surface of the particles, it is essential that the surface film, or rim of cobalt be very thin and also

14

contain an adequate loading of cobalt in the film. This is necessary to maximize reaction of the $H_2$ and CO at the surface of the particle wherein the cobalt metal reaction sites are located, while simultaneously reactions within the catalyst but outside the metal film or rim are suppressed to maximize productivity, and lower methane selectivity. The following data thus show that the catalysts of this invention, i.e., Catalysts Nos. 14-21, can be employed at productivities above 150 $hr^{-1}$, and indeed at productivities ranging above 150 $hr^{-1}$ to 200 $hr^{-1}$, and greater, to produce no more and even less methane than is produced by (i) catalysts otherwise similar except that the catalysts contain a thicker surface film, i.e., Catalysts Nos. 10-12, or (ii) catalysts which contain an insufficient cobalt metal loading within a surface film of otherwise acceptable thinness, i.e., Catalyst No. 9. The data show that the catalysts of this invention at productivities ranging above about 150 $hr^{-1}$ to about 200 $hr^{-1}$, and greater, can be employed to produce liquid hydrocarbons at methane levels well below 10 mole percent.

EXAMPLE 5 - 13

A series of twenty-one different catalysts were prepared from titania, $TiO_2$, supplied by a catalyst manufacturer in spherical form; the $TiO_2$ having the following physical properties, to wit:

14-20 Tyler mesh size (1 mm average diameter)

86-95% rutile content (by ASTM D 3720-78 test)

14-17 $m^2/g$ BET surface area

0.11-0.16 $g/cm^3$ pore volume (by mercury intrusion).

In the catalyst preparations, portions of the $TiO_2$ spheres were impregnated with cobalt nitrate and perrhenic acid via several impregnation techniques as subsequently described. In each instance, after drying in vacuo at 125-185 degrees C, the catalysts were calcined in flowing air at 250-500 degrees C for 3 hours. A first series of catalysts (Catalyst Nos. 1-8) were prepared wherein the $TiO_2$ spheres were uniformly impregnated, and these catalysts then used in a series of base runs (Table 4). Catalyst Nos. 9-11 (Table 5) and 12-21 (Table 6) were prepared such that the metals were deposited on the outside surface of the spheres to provide a shell, film or rim. The thicknesses of the catalyst rim, or outer shell, were determined in each instance by Electron Microprobe Analysis. Runs were made with these catalysts, each being contacted with synthesis gas at similar conditions and comparisons then made with those employed to provide the base runs.

Catalysts Nos. 1-8, described in Table 4 were prepared as uniformly impregnated catalysts to wit: A series of uniformly impregnated $TiO_2$ spheres were prepared by immersing the $TiO_2$ spheres in acetone solutions of cobalt nitrate and perrhenic acid, evaporating off the solutions, and then drying and calcining the impregnated spheres. The Co and Re loadings, expresses as grams metal per $cm^3$ of catalyst on a dry basis, deposited upon each of the catalysts are given the second and third columns of Table 4.

Catalysts Nos. 9-11, were prepared to contain an outer rim, or shell. These catalysts were prepared by a liquid displacement method which involves first soaking the $TiO_2$ in a water-immiscible liquid, draining off the excess liquid, and then dipping the wet spheres into a concentrated aqueous solution of cobalt nitrate (0.24g Co/ml and perrheric acid (0.02g Re/ml). Contact with the metal salt solution is limited to a very short period of time, during which the solution displaces the pre-soak liquid from the outer surface of the support particles. The rim-impregnated catalyst is quickly blotted on paper towels and dried in a vacuum oven at 140 degrees C. Results are summarized in Table 5. The second column of Table 5 thus identifies the pre-soak liquid, the third column the displacement time in minutes, the fourth and fifth columns the g $Co/cm^3$ and g $Re/cm^3$ respectively, and the sixth column the rim thickness of the outer metal shell in microns.

Catalysts Nos. 12-21, described in Table 6, were prepared to have metal shells, or rims by use of a series of spray techniques. $TiO_2$ spheres were spread out on a wire screen and preheated in a vacuum oven at various temperatures. The hot spheres were removed from the oven, sprayed with a small amount of metal salt solution, and returned without delay to the oven where drying and partial decomposition of the cobalt nitrate salt occurred. The spraying sequence was repeated several times in order to impregnate a thin outer layer, or rim of Co-Re onto the support. Preparative details are as follows:

Three solutions, I, II and III, each constituted of a different solvent, and having specific concentrations of cobalt nitrate and perrhenic acid were employed in a series of spraying procedures. The three solutions are constituted as follows:

| Solution Number | Cobalt Nitrate Concentration g/ Co/ml | Perrhenic Acid Concentration g Re/ml | Solvent |
|---|---|---|---|
| I | 0.12 | 0.01 | 20% $H_2O$ 80% Acetone |
| II | 0.12 | 0.03 | $H_2O$ |
| III | 0.12 | 0.01 | Acetone |

Five separate procedures, Procedures A, B, C, D and E, respectively, employing each of these three solutions, were employed to prepare catalysts, as follows:

A: 30 ml of Solution I added to 50 g $TiO_2$ spheres in 5 sprayings

B: 30 ml of Solution I added to 50 g $TiO_2$ spheres in 3 sprayings

C: 25 ml of Solution I added to 50 g $TiO_2$ spheres in 5 sprayings

D: 50 ml of Solution II added to 100 g $TiO_2$ spheres in 5 sprayings

E: 25 ml of Solution III added to 50 g $TiO_2$ spheres in 5 sprayings.

Reference is made to Table 6. The procedure employed in spray coating the respective catalyst is identified in the second column of said table, and the $TiO_2$ pre-heat temperature is given in the third column of said table. The $gCo/cm^3$ and $gRe/cm^3$ of each catalyst is given in Columns 4 ad 5, respectively, and the thickness of the catalyst rim is given in $\mu m$ in the sixth column of the table.

The catalysts were diluted, in each instance, with equal volumes of $TiO_2$ spheres to minimize temperature gradients, and the catalyst mixture then charged into a small fixed bed reactor unit. In preparation for conducting a run, the catalysts were activated by reduction with hydrogen at 450 degrees C, at atmospheric pressure for one hour. Synthesis gas with a composition of 64% $H_2$-32% CO-4% $N_2$ was then converted over the activated catalyst at 200 degrees C, 280 psig (1.931 MPa) for a test period of at least 20 hours. Gas hourly space velocities (GSHV) as given in each of the tables, represent the flow rate at 22 degrees C and atmospheric pressure passed over the volume of catalyst, excluding the diluent. Samples of the exit gas were periodically analyzed by gas chromatography to determine the extent of CO conversion and the selectivity to methane, expressed as the moles of $CH_4$ formed per 100 moles of CO converted. Selectivity to $C_4$ − expressed as the wt.% of $C_4$ − in the hydrocarbon product, was calculated from the methane selectivity data using an empirical correlation developed from data obtained in a small pilot plant. A productivity figure is also given for runs made with each of these catalysts, productivity being defined as the product of the values represented by the space velocity, the CO fraction in the feed and the fraction of the CO converted; the productivity being the volume CO measured at 22 degrees C and atmospheric pressure converted per hour per volume of catalyst.

Table 4

| UNIFORMLY IMPREGNATED CATALYSTS, AND GAS CONVERSION RUNS MADE THEREWITH | | | | | | | |
|---|---|---|---|---|---|---|---|
| Catalyst No | Wt.% g $Co/cm^3$ | Wt.% g $Re/cm^3$ | GHSV | %Co Conv. | Productivity | Mol.% $CH_4$ | Wt.% $C_4$ − |
| 1 | 0.0392 | 0.0034 | 200 | 67 | 43 | 5.4 | 9.4 |
| 2 | 0.0617 | 0.0046 | 750 | 50 | 120 | 10.5 | 16.9 |
| 3 | 0.1003 | 0.0080 | 500 | 80 | 128 | 11.1 | 17.7 |
| 4 | 0.0743 | 0.0056 | 750 | 64 | 154 | 11.5 | 18.3 |
| 5 | 0.0796 | 0.0050 | 750 | 71 | 170 | 13.1 | 20.7 |
| 6 | 0.1014 | 0.0084 | 750 | 77 | 185 | 13.9 | 21.8 |
| 7 | 0.0925 | 0.0066 | 750 | 77 | 185 | 13.3 | 20.9 |
| 8 | 0.1025 | 0.0068 | 1000 | 65 | 208 | 14.7 | 23.0 |

## Table 5

### RIM CATALYSTS PREPARED BY LIQUID DISPLACEMENT METHOD,

### AND GAS CONVERSION RUNS MADE THEREWITH

| Catalyst Number | Presoak Liquid | Displacement Time Mins. | g Co/cm$^3$ | g Re/cm$^3$ | Rim Thickness ($\mu$m) | GHSV | % CO Conv. | Productivity | Mol.% CH$_4$ | Wt.% C$_4$- |
|---|---|---|---|---|---|---|---|---|---|---|
| 9 | 98% Mesitylene/ 2% n-Heptanol | 2 | 0.0264 | 0.0023 | 140$^{(1)}$ | 250 | 83 | 66 | 6.5 | 11.0 |
| 10 | 98% Mesitylene/ 2%-2-Ethyl-1-hexanol | 1 | 0.0373 | 0.0031 | 200 | 500 | 66 | 106 | 7.6 | 12.6 |
| 11 | 98% Mesitylene/ 2%-2-Ethyl-1-hexanol | 2 | 0.0459 | 0.0038 | 320 | 500 | 70 | 112 | 9.3 | 15.1 |

Note (1);  The rim thickness of this catalyst, it will be noted, falls within the acceptable range. A relatively low concentration of the total metals deposited on the TiO$_2$ spheres, however, is contained within the rim.

EP 0 266 898 B1

Table 6

RIM CATALYSTS PREPARED BY SPRAYING METHOD, AND GAS CONVERSION RUNS MADE THEREWITH

| Catalyst Number | Procedure | TiO$_2$ Pre-Heat Temp. °C | g Co/cm³ | g Re/cm³ | Rim Thickness Microns | GHSV | % CO Conv. | Productivity | Mol.% CH$_4$ | Wt.% C$_4^-$ |
|---|---|---|---|---|---|---|---|---|---|---|
| 12 | A | 140 | 0.0624 | 0.0050 | 250 | 400 | 85 | 109 | 8.9 | 14.5 |
| 13 | B | 125 | 0.0818 | 0.0068 | 350 | 750 | 85 | 204 | 11.8 | 18.8 |
| 14 | C | 140 | 0.0531 | 0.0045 | 140 | 800 | 68 | 174 | 8.2 | 13.5 |
| 15 | C | 140 | 0.0613 | 0.0050 | 150 | 800 | 71 | 182 | 8.2 | 13.5 |
| 16 | C | 140 | 0.0739 | 0.0070 | 130 | 800 | 81 | 207 | 8.7 | 14.2 |
| 17 | D | 185 | 0.0507 | 0.0125 | 160 | 800 | 68 | 174 | 9.2 | 15.0 |
| 18 | E | 185 | 0.0549 | 0.0049 | 90 | 800 | 68 | 174 | 6.5 | 11.0 |
| 19 | C | 185 | 0.0483 | 0.0043 | 70 | 800 | 64 | 164 | 6.7 | 11.3 |
| 20 | C | 185 | 0.0474 | 0.0033 | 90 | 800 | 65 | 166 | 7.5 | 12.5 |
| 21 | C | 185 | 0.0603 | 0.0046 | 60 | 800 | 74 | 189 | 7.2 | 12.0 |

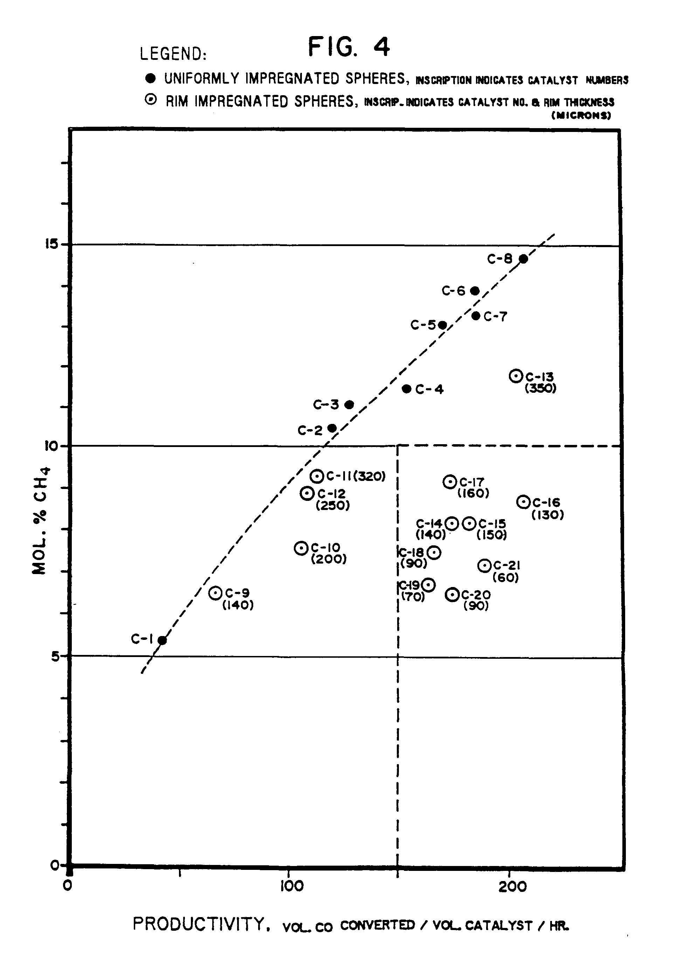

The effectiveness of these catalysts for conducting synthesis gas reactions is best illustrated by comparison of the methane selectivity at given productivity with Catalysts 1-8 (Table 4), the catalysts formed by the uniform impregnation of the metals throughout the TiO$_2$ catalyst spheres, and Catalysts 9-11 (Table 5) and 12-21 (Table 6), those catalysts wherein the metals were deposited as a shell, or rim, upon the outside of the TiO$_2$ catalyst spheres. The same type of comparison is then made between certain of the latter class of catalysts, and others, which also differ one from another dependant upon the thickness of the metals-containing rim. These data are best graphically illustrated for ready, visual comparison. Reference is thus made to Figure 4 wherein the methane selectivity produced at given productivity is plotted for each of the twenty-one catalysts described by reference to Table 4-6. A solid black data point is plotted for each of Catalysts Nos. 1-8, formed from the uniformly impregnated $TiO_2$ spheres, and each data point is identified by catalyst number. An open circle is plotted for each data point representative of Catalysts Nos. 9-21, each is identified by catalyst number, and the rim thickness of the catalyst is given. The behavior of many of these catalysts (i.e., Catalysts 9-13), it will be observed is somewhat analogous to that of Catalysts Nos. 1-8. Catalysts Nos. 14-21, however, behave quite differently from either of the other groups of catalysts, i.e., Catalysts Nos. 1-8 or Catalysts 9-13. The methane selectivity is thus relatively low for catalysts Nos. 9-13, but at the same time the productivities of these catalysts are quite low. On the other hand, the productivities of Catalysts Nos. 1-8 tend to be higher than those of Catalysts Nos. 9-13, but at the same item these catalysts produce copious amounts of methane. In striking contrast to either of these groups of catalysts, Catalysts Nos. 14-21, all of which fall within the "box" depicted on the figure, provide very high productivities and, at the same time, low methane selectivities. Catalysts Nos. 14-21 thus differ profoundly from any of Catalysts Nos. 1-13 in their behavior, and in that the metals components of these catalysts is packed into a very thin rim, or shell, on the surface of the $TiO_2$ support.

These data thus show that at constant temperature as productivity increases so too does methane selectivity for both the groups of catalysts represented by Catalysts Nos. 1-8, the uniformly impregnated catalyst, and catalysts Nos. 10-13 which have relatively thick outer shells, or rims. Thus, methane selectivity increases in proportion to the metals loadings when the metals are dispersed throughout the support, or carrier portion of the catalyst. Methane selectivity also increases in proportion to the thickness of the catalyst rim. Albeit the methane selectivities obtained with Catalysts Nos. 10-12 are within acceptable ranges, the productivities obtained with these catalysts are quite low. Catalyst No. 13 is poor on both counts. Catalyst No. 9, although it has a thin metallic rim and provides low methane selectivity, its productivity is quite poor because of an insufficient loading of metals within the rim. Catalysts Nos. 14-21 which have thin metallic rims and relatively high metals loadings within the rims, on the other hand, provide low methane selectivities and high productivities.

The results observed with Catalysts No. 1-13 are consistent with the onset of a significant diffusion limitation at the higher productivities, which intensifies as the catalysts become more active. In sharp contrast, however, catalysts which have cobalt rim thicknesses of about 180 $\mu$m, and less, notably from about 60 $\mu$m to about 160 $\mu$m, can produce at high productivities (i.e., about 150 $hr^{-1}$, or even 200 $hr^{-1}$), very low methane selectivities. Catalysts with very thin rims counteract the diffusion problem by limiting reaction to the outer surface of the catalyst wherein lies the catalytically active metal components. The catalysts of this invention thus provide a means of operating at high productivity levels with low methane selectivities. Methane selectivities are reduced at higher and higher productivities, as the rim thickness is made smaller, and smaller. When productivity is increased beyond 150 $hr^{-1}$, the metals rim should be no more than about 180 $\mu$m thick, and the rim should be even thinner. This region of operation, the best balance between activity and selectivity, is represented in Figure 4 by the area enclosed within the box formed by the dashed lines.

These data further show that the catalysts of this invention (Catalyst Nos. 14-21) can be readily prepared by the process of sequentially, or repetitively spraying hot, or preheated $TiO_2$ spheres with solutions containing compounds, or salts of the metals. Suitably, the $TiO_2$ substrate is preheated to temperatures of at least about 140 degrees C, suitably to temperatures ranging from about 140 degrees C to about 185 degrees C prior to or at the time of contact thereof with the solution. Higher temperatures can be employed, but temperatures below about 140 degrees C do not produce a sufficiently thin rim of the metals on the catalyst support. The repetitive spraying technique is shown to be superior to liquid displacement technique used to prepare Catalyst Nos. 9-11 wherein only low cobalt loadings were deposited in a single contact because of the cobalt concentration limit in the displacing solution. Longer displacement time increases the metal loading but produces a thicker rim as shown by Catalyst No. 11 compared with Catalyst No. 10. The spray technique provides especially good dispersion of the metals as a thin rim at the outer surface of the support particles by application of the metals a little at a time by multiple impregnations. Loading the metals onto the catalysts in this manner increases the activity of the catalysts, and provides higher productivity.

These reactions can be conducted with these catalysts in fixed bed, or ebullating bed reactors with or without the recycle of any unconverted gas and/or liquid product. The $C_{10+}$ product that is obtained is an admixture of linear paraffins and olefins which can be further refined and upgraded to high quality middle distillate fuels, or such other products as mogas, diesel fuel, jet fuel and the like. A premium grade middle distillate fuel of carbon number ranging from about $C_{10}$ to about $C_{20}$ can also be produced from the $C_{10+}$ hydrocarbon product. The catalyst is preferably constituted of cobalt supported on a carrier, preferably titania, and especially cobalt supported on a rutile form of $TiO_2$ or rutile-titania-containing support which can

EP 0 266 898 B1

contain other materials such as $SiO_2$, MgO, $ZrO_2$, $Al_2O_3$. The catalyst is preferably reduced with a $H_2$-containing gas at start-up.

## Claims

1. A catalyst composition useful for the conversion of synthesis gas to hydrocarbons at high productivity, with low methane selectivity, comprising cobalt dispersed as a catalytically-active layer at the surface of a support, characterised in that said layer has a thickness in the range of from 0.02 to 0.25 mm.

2. A composition according to claim 1 further characterised in that said catalytically-active layer has a thickness of up to 0.18 mm.

3. A composition according to claim 1 or claim 2 further characterised in that the catalytically-active surface layer has a thickness in the range of from 0.04 to 0.15 mm.

4. A composition according to any one of claims 1 to 3 further characterised in that the cobalt loading is in the range of from 0.01 to 0.15 $g/cm^3$, preferably from 0.03 to 0.09 $g/cm^3$, calculated as metallic cobalt per packed volume of catalyst.

5. A composition according to any one of claims 1 to 4 further characterised in that said supported catalyst is in the form of particles (e.g., beads, spheres, extrudates, rings, saddles) or pellets having a diameter in the range of from 0.5 to 2.0 mm.

6. A composition according to any one of claims 1 to 5 further characterised in that at least one metal selected from the group consisting of rhenium, hafnium, zirconium, cerium, uranium, thorium and mixtures thereof constitutes part of the catalytically-active surface layer.

7. A composition according to any one of claims 1 to 6 further characterised in that said support comprises titania.

8. A composition according to any one of claims 1 to 7 further characterised in that the support is comprised of a titania which has a rutile:anatase weight ratio of at least about 3:2.

9. A composition according to any one of claims 1 to 8 further characterised in that said layer thickness is a predetermined optimal layer thickness obtainable by calculation, for a said catalyst of productivity in excess of 150 $h^{-1}$, by relating predicted rates of diffusion of CO and $H_2$ to a predicted rate of reaction.

10. A process for the production of the catalyst of any one of claims 1 to 9 characterised by the steps comprising heating said support to a temperature equal to or in excess of 140 degrees C, and repetitively contacting said heated support with a spray of a solution which contains from 0.05 g/ml to 0.25 g/ml of a cobalt compound, calculated as metallic cobalt, per ml of solution, while drying and calcining the cobalt-containing support between each contact with the solution, to form a catalytically-active layer of cobalt at the surface of said support of average thickness in the range of from 0.02 mm to 0.25 mm, and preferably with the loading of cobalt in the range of from 0.01 $g/cm^3$ to 0.15 $g/cm^3$, calculated as metallic cobalt per packed volume of catalyst.

11. A process according to claim 10 further characterised in that the support is comprised of titania which is heated to a temperature in the range of from 140°C to 185°C and thereafter subjected to from 3 to 12 contacts with said spray solution.

12. A process for the conversion of a feedstock comprising $H_2$ and CO, such as for example a synthesis gas, to hydrocarbons, with low methane selectivity comprising contacting at reaction conditions a feed comprised of an admixture of CO and $H_2$ in a $H_2$:CO molar ratio equal to or greater than about 0.5:1 at a total (gauge) pressure equal to or greater than about 80 psig (551.5 kPa) in the presence of a catalyst composition according to any one of claims 1 to 9 or a catalyst composition made by the process of claim 10 or claim 11.

20

**13.** A process according to claim 12 further characterised in that the reaction conditions are defined within ranges as follows :

| $H_2$:CO mole ratio | | from 0.5:1 to 4:1 |
|---|---|---|
| Gas Hourly Space Velocities, Temperatures, | V/Hr,V degrees C | from 100 to 5000 from 160 to 290 |
| Total Pressure, | (psig) kPa (gauge) | (from 80 to 600) from 551.6 to 4137 |

**14.** A process according to claim 12 or claim 13 further characterised in that at productivities greater than 150 $h^{-1}$ less than 10 mol percent of the carbon monoxide which is converted is converted to methane.

**Patentansprüche**

**1.** Zur Umwandlung von Synthesegas zu Kohlenwasserstoffen mit hoher Produktivität brauchbare Katalysatorzusammensetzung mit niedriger Methanselektivität, die als katalytisch aktive Schicht auf der Oberfläche eines Trägers dispergiertes Kobalt umfaßt, dadurch gekennzeichnet, daß diese Schicht eine Dicke im Bereich von 0,02 bis 0,25 mm aufweist.

**2.** Zusammensetzung nach Anspruch 1, außerdem dadurch gekennzeichnet, daß diese katalytisch aktive Schicht eine Dicke von bis zu 0,18 mm aufweist.

**3.** Zusammensetzung nach Anspruch 1 oder Anspruch 2, außerdem dadurch gekennzeichnet, daß diese katalytisch aktive Schicht eine Dicke im Bereich von 0,04 bis 0,15 mm aufweist.

**4.** Zusammensetzung nach Anspruch 1 bis 3, außerdem dadurch gekennzeichnet, daß die Kobaltbeladung im Bereich von 0,01 bis 0,15 $g/cm^3$, vorzugsweise 0,03 bis 0,09 $g/cm^3$ liegt, berechnet als metallisches Kobalt pro gepacktem Volumen des Katalysators.

**5.** Zusammensetzung nach Anspruch 1 bis 4, außerdem dadurch gekennzeichnet, daß der Trägerkatalysator in Form von Teilchen (z. B. Perlen, Kugeln, Extrudaten, Ringen, Sattelkörpern) oder Pellets mit einem Durchmesser im Bereich von 0,5 bis 2,0 mm vorliegt.

**6.** Zusammensetzung nach Anspruch 1 bis 5, außerdem dadurch gekennzeichnet, daß mindestens ein Metall ausgewählt aus der Gruppe bestehend aus Rhenium, Hafnium, Zirkonium, Cer, Uran, Thorium und Mischungen daraus einen Teil der katalytisch aktiven Oberflächenschicht bildet.

**7.** Zusammensetzung nach einem der Ansprüche 1 bis 6, außerdem dadurch gekennzeichnet, daß der Träger Titandioxid umfaßt.

**8.** Zusammensetzung nach einem der Ansprüche 1 bis 7, außerdem dadurch gekennzeichnet, daß der Träger aus einem Titandioxid zusammengesetzt ist, das ein Rutil:Anatas-Gewichtsverhältnis von mindestens etwa 3:2 aufweist.

**9.** Zusammensetzung nach einem der Ansprüche 1 bis 8, außerdem dadurch gekennzeichnet, daß die Schichtdicke eine vorausberechnete optimale Schichtdicke ist, die durch Berechnung für den Katalysator mit einer Produktivität von mehr als 150 $h^{-1}$ erhältlich ist, indem die vorausgesagten Diffusionsgeschwindigkeiten von CO und $H_2$ mit einer vorausgesagten Reaktionsgeschwindigkeit in Beziehung gesetzt werden.

**10.** Verfahren zur Herstellung des Katalysators nach einem der Ansprüche 1 bis 9, dadurch gekennzeichnet, daß der Träger auf eine Temperatur gleich oder höher als 140°C erhitzt wird und der erhitzte Träger wiederholt mit einem Spray einer Lösung kontaktiert wird, die 0,05 g/ml bis 0,25 g/ml einer Kobaltverbindung, berechnet als metallisches Kobalt, pro ml Lösung enthält, wobei der kobalthaltige Träger zwischen jedem Kontakt mit der Lösung getrocknet und calciniert wird, um eine katalytisch

**EP 0 266 898 B1**

aktive Schicht aus Kobalt auf der Oberfläche des Trägers mit einer durchschnittlichen Dicke im Bereich von 0,02 bis 0,25 mm und vorzugsweise der Kobaltbeladung im Bereich von 0,01 bis 0,15 g/cm$^3$, berechnet als metallisches Kobalt pro gepacktem Volumen des Katalysators, zu bilden.

**11.** Verfahren nach Anspruch 10, außerdem dadurch gekennzeichnet, daß der Träger aus Titandioxid zusammengesetzt ist, das auf eine Temperatur im Bereich von 140 bis 185°C erhitzt und danach 3 bis 12 Kontakten mit der Spraylösung ausgesetzt wird.

**12.** Verfahren zur Umwandlung eines H$_2$ und CO umfassenden Einsatzmaterials, wie beispielsweise einem Synthesegas, mit niedriger Methanselektivität zu Kohlenwasserstoffen, bei dem bei Reaktionsbedingungen ein Einsatzmaterial, das aus einer Mischung aus CO und H$_2$ in einem H$_2$:CO-Molverhältnis gleich oder größer als etwa 0,5:1 zusammengesetzt ist, bei einem Gesamt(über)druck gleich oder größer als etwa 551,5 kPa (80 psig) in Gegenwart einer Katalysatorzusammensetzung gemäß einem der Ansprüche 1 bis 9 oder einer gemäß einem Verfahren nach Anspruch 10 oder Anspruch 11 hergestellten Katalysatorzusammensetzung kontaktiert wird.

**13.** Verfahren nach Anspruch 12, außerdem dadurch gekennzeichnet, daß die Reaktionsbedingungen innerhalb der folgenden Bereiche liegen:

| H$_2$:CO-Molverhältnis | | 0,5:1 bis 4:1 |
|---|---|---|
| Gasdurchsätze pro Stunde | V/h/V | 100 bis 5 000 |
| Temperaturen | °C | 160 bis 290 |
| Gesamtdruck | psig | 80 bis 600 |
| | kPa (Überdruck) | 551,6 bis 4137 |

**14.** Verfahren nach Anspruch 12 oder Anspruch 13, außerdem dadurch gekennzeichnet, daß bei Produktivitäten von mehr als 150 h$^{-1}$ weniger als 10 Mol.% des umgewandelten Kohlenmonoxids in Methan umgewandelt werden.

**Revendications**

**1.** Composition catalytique susceptible d'être utilisée dans la conversion de gaz de synthèse en hydrocarbures avec une productivité élevée et une sélectivité en méthane faible, comprenant du cobalt dispersé sous la forme d'une couche catalytiquement active à la surface d'un support, caractérisée en ce que ladite couche a une épaisseur de 0,02 à 0,25 mm.

**2.** Composition selon la revendication 1, caractérisée en outre en ce que ladite couche catalytiquement active a une épaisseur allant jusqu'à 0,18 mm.

**3.** Composition selon la revendication 1 ou 2, caractérisée par ailleurs en ce que la couche superficielle catalytiquement active a une épaisseur allant de 0,04 à 0,15 mm.

**4.** Composition selon l'une quelconque des revendications 1 à 3, caractérisée par ailleurs en ce que la charge de cobalt est de 0,01 à 0,15 g/cm$^3$, de préférence de 0,03 à 0,09 g/cm$^3$, calculée en cobalt élémentaire par volume densifié de catalyseur.

**5.** Composition selon l'une quelconque des revendications 1 à 4, caractérisée par ailleurs en ce que ledit catalyseur supporté se présente sous la forme de particules (par exemple, des perles, des sphères, des extrudats, des anneaux, des selles) ou de granules ayant un diamètre de 0,5 à 2,0 mm.

**6.** Composition selon l'une quelconque des revendications 1 à 5, caractérisée par ailleurs en ce qu'au moins un métal choisi dans le groupe constitué du rhénium, de l'hafnium, du zirconium, du cérium, de l'uranium, du thorium et de leurs mélanges contitue une partie de la couche superficielle catalytiquement active.

22

7. Composition selon l'une quelconque des revendications 1 à 6, caractérisée par ailleurs en ce que ledit support comprend du dioxyde de titane.

8. Composition selon l'une quelconque des revendications 1 à 7, caractérisée par ailleurs en ce que le support est constitué d'un dioxyde de titane qui a un rapport pondéral rutile-anatase d'au moins environ 3:2.

9. Composition selon l'une quelconque des revendications 1 à 8, caractérisée par ailleurs en ce que ladite épaisseur de couche est une épaisseur optimale prédéterminée de couche qui peut être obtenue par calcul, pour un tel catalyseur de productivité supérieure à 150 h$^-$, en rapportant les taux de diffusion prévus de CO et de $H_2$ à un taux de réaction prévu.

10. Procédé de production du catalyseur selon l'une quelconque des revendications 1 à 9, caractérisé par les stades suivants : on chauffe ledit support à une température égale ou supérieure à 140°C, et l'on met en contact de manière répétée ledit support chauffé avec un jet pulvérisé d'une solution qui contient 0,05 à 0,25 g/ml d'un composé du cobalt, calculé sur base du cobalt métallique, par ml de solution, tout en séchant et en calcinant le support contenant du cobalt entre chaque contact avec la solution, pour former une couche catalytiquement active de cobalt à la surface dudit support d'une épaisseur moyenne de 0,02 à 0,25 mm, la charge de cobalt étant de préférence de 0,01 g/cm$^3$ à 0,15 g/cm$^3$, calculée en cobalt métallique par volume densifié de catalyseur.

11. Procédé selon la revendication 10, caractérisé par ailleurs en ce que le support est constitué de dioxyde de titane qui est chauffé à une température de 140°C à 185°C et ensuite soumis à 3 à 12 contacts avec ladite solution de pulvérisation.

12. Procédé de conversion d'une charge d'alimentation comprenant du $H_2$ et du CO, notamment par exemple un gaz de synthèse, en hydrocarbures avec une faible sélectivité en méthane, le procédé comprenant la mise en contact, dans des conditions réactionnelles, d'une charge d'alimentation constituée d'une mélange de CO et de $H_2$ selon un rapport molaire $H_2$:CO égal ou supérieur à environ 0,5:1 sous une pression (manométrique) totale égale ou supérieure à 551,5 kPa (80 psig) en présence d'une composition catalytique selon l'une quelconque des revendications 1 à 9 ou d'une composition catalytique préparée selon le procédé de la revendication 10 ou 11.

13. Procédé selon la revendication 12, caractérisé par ailleurs en ce que les conditions réactionnelles sont définies dans les plages suivantes :

| | |
|---|---|
| Rapport molaire $H_2$:CO | de 0,5:1 à 4:1 |
| Vitesses spatiales horaires du gaz V/h, V | de 100 à 5000 |
| Températures °C | de 160 à 290 |
| Pression totale kPa (man.) (psig) | de 551,6 à 4137 (de 80 à 600) |

14. Procédé selon le revendication 12 ou 13, caractérisé par ailleurs en ce qu'à des productivités supérieures à 150 h$^{-1}$, une proportion de moins de 10 moles pour-cent du monoxyde de carbone qui est convertie l'est en méthane.

FIG-1

6% CO-0.5% Re CATALYST
T = 200°C
$(P_{H_2}/P_{CO})_{feed}$ = 2.0
$P_t$ = 19. atm

METHANE SELECTIVITY,%

PELLET RADIUS, mm

EP 0 266 898 B1

FIG-2

FIG-3

EP 0 266 898 B1

LEGEND:

FIG. 4

● UNIFORMLY IMPREGNATED SPHERES, INSCRIPTION INDICATES CATALYST NUMBERS

⊙ RIM IMPREGNATED SPHERES, INSCRIP. INDICATES CATALYST NO. & RIM THICKNESS (MICRONS)

27